# FASCICULE DE BREVET EUROPEEN

(11) **EP 2 020 954 B1**
(45) Date de publication et mention de la délivrance du brevet: **04.09.2013**
(21) Numéro de dépôt: 06764695.0
(22) Date de dépôt: 24.05.2006
(51) Int. Cl.: A61D 7/04

(54) **CELLULE UNIVERSELLE POUR L'IMAGERIE MÉDICALE D'UN PETIT ANIMAL SOUS ANESTHÉSIE**
UNIVERSALZELLE ZUR MEDIZINISCHEN BILDDARSTELLUNG EINES KLEINEN TIERES UNTER NARKOSE
UNIVERSAL CELL FOR MEDICAL IMAGING OF A SMALL ANIMAL UNDER ANESTHESIA

(43) Date de publication de la demande: 11.02.2009
(73) Titulaire: Equipement Veterinaire Minerve, 51310 Esternay (FR); Sannie, Vincent, 51210 Corfelix (FR); Sannie Sebastien, 77120 Marolles en Brie (FR)
(72) Inventeur: SANNIE, Vincent, F-51210 Corfelix (FR); SANNIE, Sebastien, F-77120 Marolles en Brie (FR)
(74) Mandataire: Intès, Didier Gérard André
(86) Numéro de dépôt international: PCT/FR2006/001218
(87) Numéro de publication internationale: WO 2007/135248

(56) Documents cités:
- US-A- 5 099 792
- US-A- 5 297 502
- US-B1- 6 776 158

## Description

L'invention s'inscrit dans le domaine des activités de recherches liées aux techniques d'investigation en imagerie médicale du petit animal de laboratoire telles que l'imagerie par résonance magnétique, la microtomodensitométrie par rayon X, les techniques d'exploitation scintigraphiques par émetteurs monophotoniques et biphotoniques, l'imagerie optique . L'acquisition des images pouvant s'étaler sur plusieurs heures , les animaux contaminés ou non doivent être maintenus calmes, insensibles à la douleur et immobiles. Cet état physiologique est caractérisé par une bonne anesthésie générale chimique ou volatile, une bonne ventilation et un bon maintien de la température corporelle de l'animal.

L'invention , réalisée en matériaux amagnétiques, est constituée de deux dispositifs dont l'un assure la pré-anesthésie et l'autre le maintien sous anesthésie volatile prolongée de l'animal de laboratoire ainsi que son isolation de l'air ambiant tout en permettant un traitement médical par voie sanguine ou respiratoire. De plus, ce dernier dispositif , contenant l'animal sous traitement , est destiné à être transféré, manuellement ou par système robotisé , puis inséré dans un imageur pour les acquisitions d'images médicales .

### ETAT DE LA TECHNIQUE ANTERIEURE

Pour assurer la pré-anesthésie d'un animal, il existe des cages ou chambres d'induction qui ont pour point commun :
- l'obligation du remplissage du volume disponible de gaz anesthésiants suivie par l'obligation de vidange du même volume avant d'extraire l'animal pour les suites à donner .
- elles sont fortement consommatrices en agents anesthésiques , gaz vecteur et autres consommables destinés à capter les vapeurs polluantes extraites .
- elles exigent une manipulation contraignante, une immobilisation de l'animal relativement longue et un temps global de traitement important (à paramètres d'induction constants, selon le modèle de cage et le fabricant, selon les caractéristiques physiologiques de l'animal, entre 2 et 4 minutes)
- elles exigent la mise en place de systèmes de capture des gaz halogénés en surplus afin de préserver les utilisateurs de l'exposition aux halogénés .
Cependant, elles n'évitent pas l'imprégnation de la fourrure de l'animal par les vapeurs halogénées dont les particules s'évaporeront directement dans l'environnement de travail des utilisateurs

Pour assurer le maintien sous anesthésie volatile prolongée de l'animal, les systèmes existants sont conçus selon le principe des circuits à double canalisations avec induction des gaz anesthésiques / extraction simultanée des gaz polluants ou de surpression. Dans leur forme actuelle , certains systèmes , assurant la fonction d'endormissement et de maintien sous anesthésie volatile , répondent à beaucoup d' applications de recherches. Selon le modèle et le fabricant, ils peuvent être simples ou permettre le réchauffement de l'animal afin d'éviter l'hypothermie et ses conséquences.

Cependant, les systèmes existants ne sont ni magnétiques , ni destinés à être transférés manuellement ou automatiquement , ni insérables dans un imageur de sorte que le besoin d'investigations en imagerie médicale ne serait pas satisfait.

US 5 099 792, US 5 297 502 et US 6 776 158 décrivent des dispositifs pour anesthesier des animaux tels que des rats ou des souris.

### EXPOSE DE L'INVENTION

L' invention traite l'aspect matériel en aval d'une source de distribution de gaz anesthésiants et d'un système d'extraction dynamique des gaz halogénés de surpression.

Il s'agit d'un dispositif conçu pour traiter dans son ensemble , la fonction endormissement d'un animal de laboratoire et la fonction anesthésie volatile prolongée avec positionnement précis et reproductible de son corps , son réchauffement, son isolement de l'air ambiant , son traitement médical par voie sanguine ou respiratoire ; de sorte que des investigations en imagerie médicale sur l'animal à étudier pourront être entreprises.

Un tel dispositif est défini par la revendecation 1.

La cellule d'isolement transférable permet :
- le maintien sous anesthésie prolongée de l'animal à étudier durant plusieurs heures
- la mise à disposition d'un espace disponible maximal pour l'animal tout en respectant le besoin d'encombrement minimal de la cellule à insérer dans un imageur
- l'isolement de l'animal , contaminé ou non , de l'air ambiant et des utilisateurs
- la mise en place , au moyen d'un accès sécurisé allant de l'extérieur vers l'intérieur de la cellule d'isolement , de divers accessoires permettant la surveillance des paramètres physiologiques de l'animal anesthésié et d'insuffler par séquences , divers mélanges gazeux spécifiques à l'imagerie médicale (gaz hyperpolarisés)
- de juguler la baisse de la température corporelle de l'animal, qu'elle soit spontanée ou inhérente à l'anesthésie.
- d' assurer un positionnement précis et reproductible du corps de l'animal à étudier
- le transfert , manuel ou robotisé , de la cellule d'isolement occupée par l'animal vers les appareils dédiés aux modalités d'investigations en imagerie médicale telles que:
   * l'imagerie par résonance magnétique (IRM-MRI)
   * la microtomodensitométrie par rayon X (micro scanner X, micro CT)
   * les techniques d'exploitation scintigraphiques par émetteurs monophotoniques (micro gamma camera , micro TEMP, micro SPECT) et biphotoniques ( microTEP, micro PET)
   * l'imagerie optique du petit animal (émetteurs fluorescents)
- de satisfaire à la sécurité des utilisateurs , au moyen des prises de raccordement sécurisées à double obturation qui équipent tous les postes et accessoires.

En ce qui concerne le dispositif d'induction : C'est un accessoire au générateur de gaz anesthésiants permettant l'endormissement de l'animal à traiter. Il y est relié au moyen d'un circuit coaxial permettant la circulation de deux flux de gaz en simultané. L'un provenant du générateur vers le dispositif, l'autre inversement provenant du dispositif vers une unité d'aspiration des gaz polluants résiduels. Ce circuit est pourvu aux deux extrémités d'une prise mâle avec système à double obturation. Ces prises sont conçues pour se raccorder à une prise femelle , elle-même pourvue du système à double obturation .Si les prises male/femelle à double obturation sont désolidarisées l'une de l'autre, les flux d'arrivée des halogénés et d'extraction des gaz pollués résiduels seront obturés. Cet équipement permet d'isoler par rapport à l'environnement le circuit coaxial lui même mais aussi tous les dispositifs qui y sont raccordés. De plus il n'y aura aucune émission de gaz halogénés dans l'environnement si le circuit coaxial, - raccordé au générateur de gaz anesthésiants - , ne l'est pas au dispositif d'induction ou à la cellule d'isolement.

Les prises male/femelle à double obturation sont conçues de façon à activer, au moment du couplage , l'extraction des gaz résiduels dans un premier temps puis l'induction des anesthésiants dans un deuxième temps de sorte que toute fuite d'halogénés, au moment du raccordement , serait captée. Elles constituent une sécurité de branchement des matériels périphériques au poste d'anesthésie. Ce système de prises mâles/femelles sécurisées équipent le générateur de gaz anesthésiants , le dispositif d'induction et la cellule d'isolement et fait partie intégrante de l'invention.

Le dispositif d'induction est constitué d'un corps principal fixé sur un bâti assurant la stabilité mécanique de l'ensemble, et de deux parties mobiles solidarisables constituées par le cylindre d'induction et le corps mobile. Le cylindre d'induction est équipé , au niveau de son extrémité postérieure d'une partie conique de dimensions adaptées à l'espèce animale à contenir et à induire. L'extrémité antérieure est montée en liaison pivot par rapport au bâti. Le débattement angulaire de cette liaison pivot est limité à 90 ° de sorte que le cylindre d'induction peut débattre d'une position verticale à une position horizontale. En position horizontale, le cylindre d'induction permet la mise en place , tête la première , de l'animal vif . Le passage de la position horizontale à la position verticale est réalisé manuellement par l'utilisateur du dispositif . Cette manoeuvre actionne l'ouverture mécanique d'un robinet situé dans le corps du bâti. Elle autorise le passage des gaz halogénés via un canal qui débouche dans la partie antérieure du cylindre et aboutit précisément au museau de l'animal. Les effets de l'induction sont pratiquement immédiats du fait de l'absence de volume mort à saturer. La distribution de gaz anesthésiants pourra rester très faible soit environ un volume égale au produit du volume courant par la fréquence respiratoire de l'espèce animale concernée (V = VC x Fréq ). Le cylindre d'induction est équipé d'une aspiration permanente située en amont de la source de distribution des gaz anesthésiants. Cette aspiration est en service quelle que soit la position du cylindre, (verticale ou horizontale) de façon à capter au plus près possible de la source d'émission les gaz halogénés en excès. La partie centrale du cylindre , munie d'une fenêtre transparente, permet à l'utilisateur de visualiser le comportement de l'animal à induire. A l'issue du temps d'induction nécessaire soit environ 20 à 30 secondes d'exposition, l'utilisateur interrompt l'envoi des gaz anesthésiques en basculant le cylindre de la position verticale à la position horizontale. L'animal endormi , dont le pelage est préservé des vapeurs halogénées , peut être extrait du cylindre d'induction. Pour se faire, l'opérateur désolidarise le cylindre de son bâti et accède ainsi à la partie antérieure de l'animal. Il pourra être aisément dégagé et positionné sur un accessoire de maintien standard sous anesthésie ou dans la cellule d'isolement en position ouverte. Pendant cette période de manipulation , l'aspiration est toujours active et évacue les particules halogénées résiduelles.

Ce dispositif offre un gain de temps important (de l'ordre de 5 à 6 fois plus rapïde que la cage à induction), une sécurité accrue des utilisateurs (les pelage des animaux manipulés est préservé des vapeurs halogénées). Les coûts de revient des expérimentations sont donc réduits au niveau des consommations d' agent anesthésique , de gaz vecteur, d'éléments filtrants.

En ce qui concerne la cellule d'isolement transférable: C'est également un accessoire au générateur de gaz anesthésiants. Elle permet de maintenir sous anesthésie gazeuse un animal destiné à être inséré dans un imageur. Elle est reliée au générateur au moyen d'un circuit coaxial permettant la circulation de deux flux de gaz en simultané L'un provenant du générateur de gaz anesthésiants vers le dispositif, l'autre, inversement, provenant du dispositif vers une unité d'aspiration des gaz pollués résiduels. Il s'agit du même type de circuit que celui utilisé avec le dispositif d'induction.

La cellule d'isolement est constituée d'un corps principal et d'une partie mobile avec bulle isolante. L'ensemble repose sur un bâti fixe assurant la stabilité mécanique du produit. Désolidarisée de son bâti fixe, la cellule d'isolement contenant l'animal , peut être transférée manuellement ou par système robotisé vers une unité d'acquisition de données en imagerie médicale.

Le corps principal est pourvu de deux canaux indépendants permettant , pour l'un, la circulation du gaz froid extrait de la cellule d'isolement , pour l'autre, la circulation retour de ce gaz qui sera réintroduit après avoir été retraité par le système de réchauffe . Ces deux canaux indépendants son connectés d'un côté aux canalisations d'un lit réalisé en profilé extrudé et de l'autre à un échangeur thermique intégré au corps principal. Le système de chauffe, fonctionnant en boucle fermée, est composé d'une pompe d'extraction de gaz qui, connectée à une unité de régulation de température et à l'échangeur thermique du corps , permet la réchauffe des gaz froids extraits de sorte qu'il seront réintroduits dans la cellule à une température suffisante pour que l'animal , contenu sur le lit, conserve ou récupère la température corporelle voulue. Dans ce corps principal s'emboîte un tunnel de maintien avec un canal technique , avec ou sans membrane perforable , permettant le passage de cathéters, sondes, électrodes, circuits respiratoires, circuits pneumatiques de commande ou autre. La partie mobile est constituée d'un lit support et d'un contenant externe transparent. Le lit , réalisé en profilé extrudé avec canaux longitudinaux utilisés pour le passage de divers fluides à véhiculer (induction/extraction des gaz anesthésiants, fluide caloporteur ...) . Ce lit s'emboîte dans le tunnel de maintien (lui même emboîté dans le corps principal) . L'ensemble de la partie mobile permet la contention de l'animal ainsi que son isolation de l'air ambiant lorsque ce contenant externe est vissé sur la molette de réglage d'aspiration des gaz disposée sur le corps principal. Les pièces de cette partie mobile peuvent être constituées d'éléments stériles à usage unique pour les applications interdisant tout risque de contamination croisée. La cellule peut être utilisée en position ouverte (absence d'isolement de l'animal) ou fermée (avec isolement de l'animal) si son contenant externe transparent est vissé à la molette de réglage de puissance d'aspiration des gaz de surpression contenus dans la cellule.

La position ouverte est obtenue en tournant la molette de commande vers la position induction+extraction . Elle permet à l'utilisateur de positionner/contenir/préparer l'animal qui vient d'être extrait du dispositif d'induction et le disposer sur un lit aux formes et dimensions adaptées à sa morphologie. Ce lit , réglable en rotation autour de l'axe longitudinal, avec repères gradués longitudinaux et barres d'oreilles pour la contention stéréotaxique, permet un positionnement précis et reproductible de l'animal dans l'imageur. Le museau de l'animal est dirigé vers le cône avec pièce de dent du tunnel de maintien assurant son immobilisation et l'admission directe des halogénés. En périphérie de ce cône et en aval de son museau, sont disposés des canaux d'extraction permettant l'aspiration maximale des gaz pollués en surplus. Après la mise en place de l'animal sous anesthésie , sa connexion aux matériels adaptés au protocole expérimental via l'accès sécurisé allant de l'extérieur vers l'intérieur de la cellule, sa connexion au système de réchauffe, l'utilisateur peut transférer directement l'animal réchauffé vers l'imageur dans la cellule en position ouverte ou mettre en place la bulle isolante si l'animal doit être isolé de l'air ambiant.

La position fermée est obtenue en vissant la bulle transparente sur la molette de commande de réglage de puissance d'aspiration des gaz de surpression puis en tournant complètement la molette de commande vers la position induction+surpression . Cette molette sera actionnée après la fermeture de la bulle transparente. La mise en service de cette fonction a pour conséquence de laisser le passage des halogénés vers l'animal alors que l'extraction sera déviée mécaniquement de sorte que l'intérieur de la cellule ne sera que partiellement aspiré. La déviation mécanique de l'extraction évite ainsi une dépression à l'intérieur de la cellule. L'animal , maintenu sous anesthésie , réchauffé, isolé du milieu ambiant , traité selon les besoins de l'application, est prêt à être transféré manuellement ou par système robotisé , de la zone de préparation vers la zone d'investigation en imagerie médicale , ou à être mis en attente de traitement ultérieur. En phase finale d'expérimentation, l'animal sera retiré de la cellule qui pourra être réutilisée après décontamination et/ou ré-équipée d'autres éléments stériles ou nom.

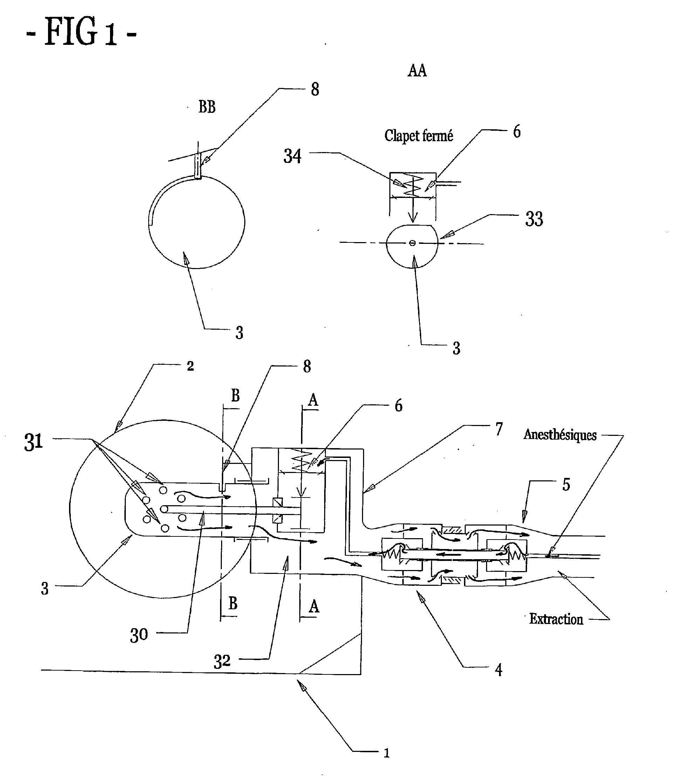

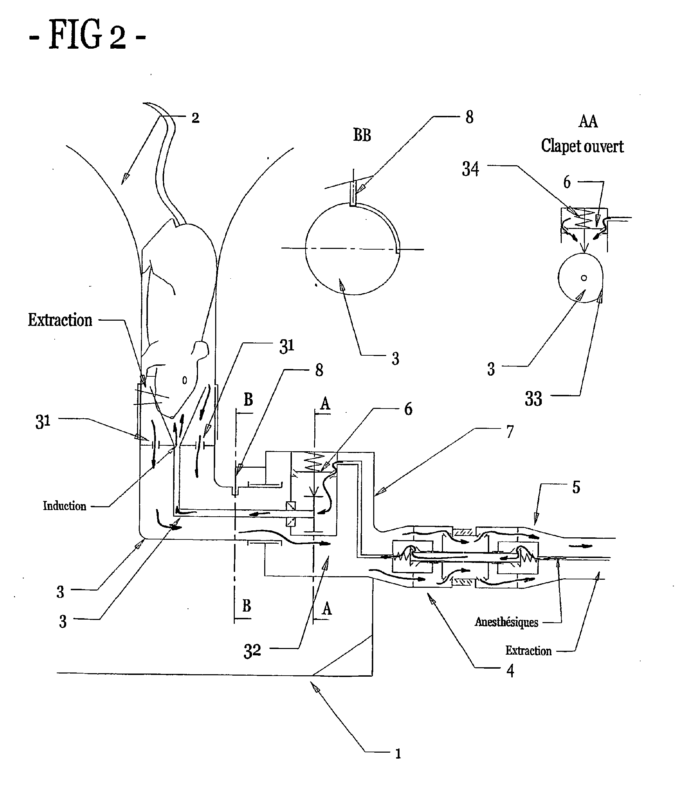

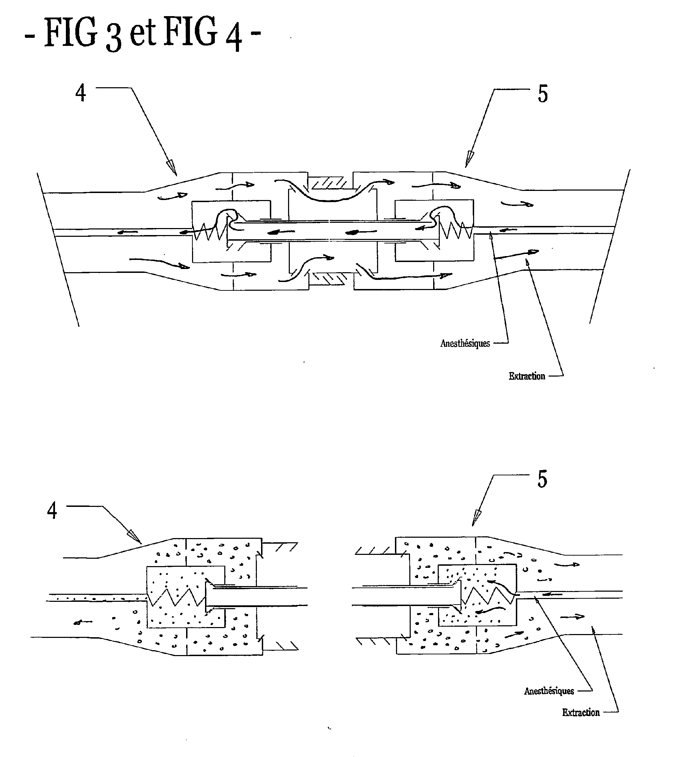

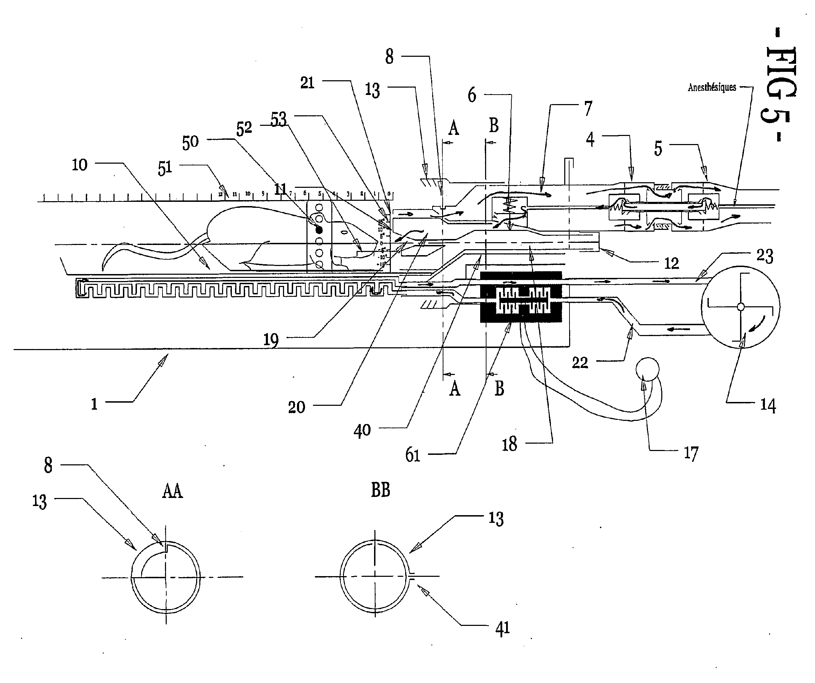

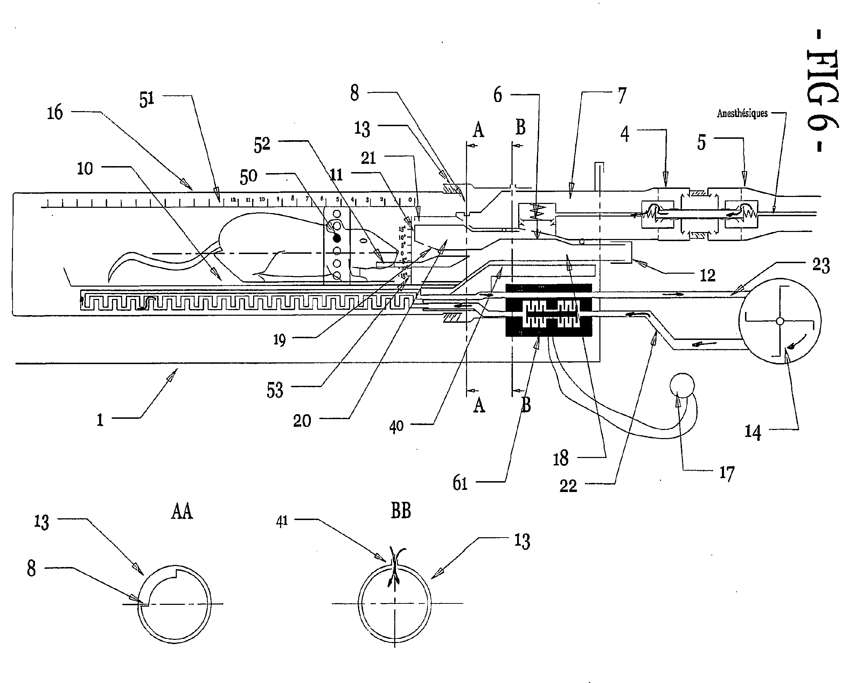

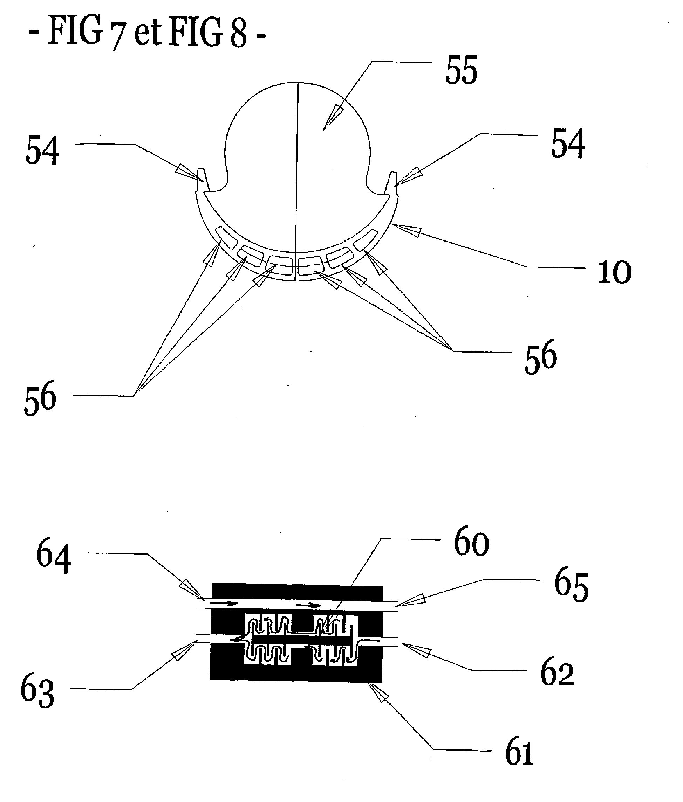

PRESENTATION SYNTHETIQUE DES 8 SCHEMAS DES ANNEXES 1 A 6 constituant le dispositif d'induction (Fig 1 et 2 ), le système sécurisé de prises male/femelle à double obturation (Fig 3 et 4) et la cellule d'isolement transférable (Fig 5, 6,7et8)
- 1/ 8 Schéma de principe du dispositif induction CYLINDRE HORIZONTAL (Fig 1)
   - Coupe AA visualisation du clapet fermé
   - Coupe BB visualisation de l'arrêt en rotation du cylindre -2/8 Schéma de principe du dispositif induction CYLINDRE VERTICAL (Fig 2)
   - Coupe AA visualisation du clapet ouvert
   - Coupe BB visualisation de l'arrêt en rotation du cylindre
- 3/ 8 Schéma de principe prises mâle/femelle CONNECTEES (Fig 3)
- 4/8 Schéma de principe prises mâle/femelle DECONNECTEES Fig 4)
- 5/8 Schéma de principe de la cellule non isolée POSITION OUVERTE (Fig 5)
   - Coupe AA visualisation de l'arrêt en rotation de la molette
   - Coupe BB visualisation de la prise d'air obturée
- 6/ 8 Schéma de principe de la cellule isolée POSITION FERMEE (Fig 6)
   - Coupe AA visualisation de l'arrêt en rotation de la molette
   - Coupe BB visualisation de la prise d'air ouverte
- 7/ 8 Schéma en coupe d'un modèle de lit avec canaux longitudinaux permettant le passage de plusieurs fluides et la fixation d'accessoires par clips (Fig 7)
- 8/8 Schéma en coupe de l'échangeur thermique inséré dans le corps principal (Fig 8)

### EXPOSE DETAILLE D'UN MODE DE REALISATION se référant aux schémas Fig 1 à 8

Figures 1 et 2 : Le dispositif d'induction est lié en permanence à un bâti fixe(1) posé sur une paillasse ou plan de travail. Ce bâti assurant la stabilité de l'ensemble mécanique est de dimension, de forme, de poids tels qu'il reste stable lors de la phase d'induction. Le dispositif d'induction comprend un corps principal (7) fixé sur le bâti , avec prise coaxiale femelle à double obturation (4) intégrée et de deux parties mobiles solidarisables. La première est constituée du cylindre d'induction et de contention (2) la seconde du corps mobile (3). La prise coaxiale femelle (4) équipant le corps principal (7) doit être connectée (Fig 3) et vissée à la prise coaxiale mâle à double obturation(5) du circuit coaxial raccordant le générateur de gaz anesthésiant au dispositif d'induction afin de permettre deux circulations libres et indépendantes de gaz . La première véhicule les gaz halogénés du générateur de gaz anesthésiant vers le dispositif d'induction . La deuxième extrait les gaz halogénés résiduels du dispositif d'induction vers l'unité d'aspiration des gaz pollués située en aval .

Si la prise coaxiale femelle (4) est déconnectée (Fig 4) de la prise mâle, (5), les deux flux de gaz seront obturés de sorte qu'il n'y aura aucune émission de gaz halogénés ni aspiration de l'air ambiant de l'environnement de travail. De plus, les systèmes situés en amont et en aval des prises femelle/mâle seront complètement isolés .

La partie mobile appelée cylindre d'induction et de contention (2) est de forme et de dimension adaptées à la morphologie de l'animal à induire . Ce cylindre d'induction, muni d'une fenêtre de surveillance, est pourvu d'un perçage central permettant le libre passage des halogénés jusqu'au museau de l'animal à induire et de canaux périphériques (31) permettant la libre extraction des halogénés résiduels. Le cylindre d'induction (2) peut être rapidement désolidarisé du corps mobile (3) lors des opérations de changement de cylindre de plus ou moins grande taille par déboîtement.

Le corps mobile (3) est monté en liaison pivot sur le corps principal (7) avec une rotation limitée à 90 ° de façon à ce que le cylindre d'induction (2) puisse débattre de la position horizontale (Fig 1) vers la position verticale (Fig 2). Son arrêt en rotation et translation par rapport au corps principal (7) est assuré par un pion d'arrêt (8).

Le corps mobile (3) est pourvu d'un perçage (32) permettant de faire communiquer les canaux d'aspiration (31) du cylindre d'induction (2) au perçage dédié du corps principal (7) quelle que soit la position (horizontale Fig 1 ou verticale Fig 2) du cylindre d'induction (2) de sorte que l'extraction des halogénés résiduels est toujours active.

L'extrémité du corps mobile (3) est munie d'une came (33) permettant l'ouverture ou la fermeture des gaz halogénés . Le clapet (6) selon qu'il est actionné (Fig 2 - Coupe AA) ou pas (Fig 1 - Coupe AA) est décollé du corps principal (7) ou pas. Il a donc pour fonction d'ouvrir ou fermer l'arrivée des gaz halogénés. Lorsque le cylindre d'induction (2) est en position horizontale (Fig 1) les gaz halogénés sont stoppés au niveau du clapet (6) qui est maintenu fermé et en contact avec le corps principal (7) à l'aide du ressort (34). Cette position permet la mise en place de l'animal vif. Lorsque le cylindre d'induction (2) est en position verticale (Fig 2), les gaz halogénés peuvent franchir le clapet (6) ouvert et décollé du corps principal (7), traverser le corps mobile (3) et le canal central et déboucher au centre du cylindre d'induçtion . Cette position permet à l'animal vif de passer au stade d'endormissement . L'état de fonctionnement de l'appareillage est repéré sur le corps mobile (3) :
Position verticale du cône d'induction: Extraction+induction (animal en cours d'induction) Position horizontale du cône d'induction: Extraction (en attente d'animal à induire ou arrêt)

Figures 5 , 6 , 7 et 8 : La cellule d'isolement est supportée par un bâti indépendant (1) , lui-même posé sur une paillasse ou un plan de travail . Ce bâti (1) assurant la stabilité de l'ensemble mécanique est de dimension, de forme, de poids- tels qu'il reste stable pendant toute la phase de préparation de l'animal préalablement induit. La cellule d'isolement comprend un corps principal (7) avec prise coaxiale femelle à double obturation (4), une molette de réglage (13), et trois parties amovibles . La première : le lit (10) sur lequel l'animal induit est mis en place , la seconde : tunnel de maintien (11) avec cône (19) et pièce de dent (52), la troisième : le contenant externe transparent (Fig 6 - 16) . L'ensemble monté se désolidarise du bâti (1) de sorte qu'il peut être transféré vers un imageur.

La prise coaxiale femelle (4) équipant le corps principal (7) doit être connectée (Fig 3) et vissée à la prise coaxiale mâle à double obturation (5) du circuit coaxial raccordant le générateur de gaz anesthésiant à la cellule afin de permettre deux circulations libres et indépendantes de gaz . La première véhicule les gaz halogénés du générateur de gaz anesthésiant vers la cellule d'isolement , la deuxième extrait les gaz pollués résiduels de la cellule d'isolement vers l'unité d'aspiration des gaz pollués située en aval . Si cette prise coaxiale femelle (4) est déconnectée (Fig 4) de la prise mâle (5), les deux flux de gaz seront obturés de sorte qu'il n'y aura aucune émission de gaz halogénés ni aspiration de l'air ambiant de l'environnement de travail. De plus, les systèmes situés en amont et en aval des prises femelle/mâle (4 - 5) seront complètement isolés.

Le corps principal (7) est équipé de deux canaux et d'un perçage (40) réservé au passage du tunnel de maintien (11) et à son emboîtement . Ces deux canaux disposent de sorties à connecter d'un côté au système de réchauffe fonctionnant en boucle fermée comprenant une pompe (14) d'extraction des gaz froids , une unité de régulation de température (17), un échangeur thermique (61 Fig 5 et 6 et Fig 8) intégré au corps principal (7) ; de l'autre côté aux canaux longitudinaux (56- Fig 7)) du lit (10). Ainsi, les gaz froids (64 - 65 Fig 8/8) extraits par la pompe (14 - Fig 5 et 6) des canaux longitudinaux du lit (56 - Fig 7) traversent l'élément chauffant (60) via les canaux (62 - 63 Fig 8) de l'échangeur , se chargent en calories, et sont restitués réchauffés dans les canaux longitudinaux (56 - Fig 7 et 5-6) du lit (10) . Le canal (41) du corps principal(7) permet de faire communiquer vers l'extérieur l'aspiration provenant du cône(19).

La première partie amovible appelé lit (10) est de forme et de dimension adaptées à la morphologie de l'animal à traiter. Ce lit (10), équipé de barre d'oreille (50) et de repères gradués (51) est solidaire du tunnel de maintien (11) lui-même équipé d'une pièce de dent (52) et se désolidarise par simple traction. Il est réalise dans un profilé extrudé (Fig 7) permettant d'intégrer dans son épaisseur , des canaux longitudinaux (56) utilisés pour le passage des divers fluides à véhiculer (induction / extraction des gaz anesthésiants, fluide caloporteur pour thermorégulation de l'animal contenu sur le lit). Ce profilé , en arc de cercle, de quelques millimètres d'épaisseur et de diamètre adapté est muni de griffes (54) sur lesquelles pourront se clipper divers couples supports amovibles (55) pour accessoires. En cas d'utilisation interdisant tout risque de contamination croisée le lit peut être stérilisé ou à usage unique .

La deuxième partie amovible appelée tunnel de maintien (11) est pourvue d'un cône (19) équipé d'une pièce de dent (52) sur laquelle sont positionnées les incisives de l'animal à traiter. Dans le prolongement du tunnel de maintien (11) existe un canal technique (18) obturé ou non par un bouchon (12) . Ce bouchon (12) perforàblè assure un accès sécurisé de l'extérieur vers l'intérieur de la cellule . Le passage ainsi dégagé permet la mise en place de cathéter , sonde, électrode... abouchant directement au niveau du museau de l'animal . Ces divers équipements seront mis en place lors de la phase de sa préparation. Le tunnel de maintien (11) est également pourvu d'un canal (20) dédié au passage des gaz halogénés directement délivrés au niveau du museau de l'animal et de canaux périphériques (21) permettant l'aspiration des gaz halogénés résiduels. Lorsque le tunnel de maintien (11) est complètement emboîté dans le corps principal (7), il actionne le clapet (6) et permet le passage des gaz halogénés . Lorsque le tunnel de maintien (11) est enlevé du corps principal (7), le clapet (6)obture le passage des gaz halogénés. Le tunnel de maintien (11) est orientable en inclinaison suivant son axe longitudinal. Le tunnel de maintien (11) est muni de repères (53) permettant de connaître l'angle d'inclinaison décrit entre le corps principal (7) et le tunnel (11) . Cette fonctionnalité permet d'orienter l'ensemble lit (10) + animal+tunnel (11). Le tunnel de maintien (11) peut être rapidement désolidarisé par simple traction et à usage unique comme le lit (10) en cas de risque de contamination croisée

La troisième partie amovible appelée contenant externe (16) , de forme et de dimension adaptées à la morphologie de l'animal , transparent et de faible épaisseur , est visée sur la molette de réglage (13) à l'aide d'un filetage pour fermeture rapide. Sa mise en place isole complètement l'animal à traiter de l'air ambiant. La molette de réglage (13) est montée en liaison pivot (fig 6 coupe AA) sur le corps principal (7) avec une rotation limitée à 90° par rapport à l'axe longitudinal de la cellule. Son arrêt en rotation et translation par rapport au corps principal (7) est assuré par un pion d'arrêt (8). La molette (13) se règle selon deux positions distinctes :
1°) position cellule ouverte (Fig 5) - induction+extraction : extraction maximale puisque le canal d'aspiration est complètement libre - animal anesthésié, réchauffé mais non isolé
2°) position cellule fermée (Fig 6) - induction+surpression : extraction minimale puisque la prise d'air (41) située à l'extérieur de la cellule accentue les effets de la réduction d'aspiration . Lorsque la cellule est fermée, l'extraction est utilisée comme dispositif de collecte des gaz halogénés résiduels - animal anesthésié , réchauffé et isolé de l'air ambiant

## Revendications

1. Dispositif dédié aux applications liées aux techniques d'investigations en imagerie médicale du petit animal de laboratoire telles que l'imagerie par résonance magnétique, la microtomodensitométrie par rayon X, les techniques d'exploitation scintigraphiques par émetteurs monophotoniques et biphotoniques, l'imagerie optique le dispositif comprenant :
- un système d'induction directe de gaz anesthésiant au niveau de son museau préservant ainsi son pelage du gaz anesthésiant et permettant sa pré-anesthésie, le système d'induction directe de gaz anesthésiant comportant deux parties mobiles (2, 3) et une partie fixe, appelée corps principal (7), solidarisée à un bâti (1), l'une des partie mobile (2), appelée cylindre d'induction, pouvant se désolidariser de la deuxième partie mobile (3) par déboîtement, et
- une cellule d'isolement transférable assurant le positionnement très précis et reproductible du corps de l'animal et son isolement de l'air ambiant lorsqu'elle est fermée ladite cellule d'isolement transférable permettant le maintien de l'animal sous anesthésie prolongée, son réchauffement, son traitement médical par voie sanguine ou respiratoire de sorte que ladite cellule d'isolement transférable contenant l'animal sous traitement, utilisée ouverte ou fermée, peut être inséré manuellement ou par système robotisé, vers des imageurs pour les acquisitions d'images médicales, **caractérisé en ce que** la deuxième partie mobile (3), appelée corps mobile, est montée en liaison pivot sur le corps principal (7), de sorte que le cylindre d'induction (2) peut débattre depuis une position horizontale vers une position verticale, la deuxième partie mobile (3) étant pourvu d'un passage commandé et réservé à l'induction de gaz anesthésiant permettant de faire communiquer l'induction de gaz anesthésiant du corps principal (7) à un canal central (30) du cylindre d'induction (2) de sorte que l'induction de gaz anesthésiant est activée lors du passage du cylindre d'induction (2) de la position horizontale à la position verticale, la cellule d'isolement transférable comprend une première partie amovible appelée lit (10), réalisée en profilé extrudé permettant d'intégrer dans son épaisseur des canaux longitudinaux (56) utilisés pour le passage de divers fluides à véhiculer, tels que des gaz anesthésiants et/ou un fluide caloporteur pour la thermorégulation de l'animal contenu sur le lit (10).

2. Dispositif selon la revendication 1 **caractérisé en ce que** le système d'induction directe de gaz anesthésiant et la cellule d'isolement transférable sont des accessoires d'un générateur de gaz anesthésiant et d'un système d'extraction des gaz pollués et y sont reliés au moyen d'un circuit coaxial qui permet la circulation libre et indépendante de deux gaz dont l'une est dédiée à l'induction des gaz anesthésiants et l'autre à l'extraction des gaz pollués.

3. Dispositif selon la revendication 2 **caractérisé en ce que** le système d'induction directe de gaz anesthésiant et la cellule d'isolement transférable disposent d'une prise de raccordement femelle (4) à double obturation à connecter à une prise de raccordement mâle (5) à double obturation du circuit coaxial de sorte que la circulation libre et indépendante de deux gaz s'effectue si les deux prises sont connectées alors qu'elle est interdite et isole le système d'induction directe et la cellule d'isolement transférable si les deux prises mâle (5) et femelle (4) sont déconnectées.

4. Dispositif selon l'une quelconque des revendications 1 à 3 **caractérisé en ce que** la première partie mobile (2) est munie de canaux périphériques d'aspiration (31) permettant la libre extraction des gaz anesthésiants résiduels.

5. Dispositif selon la revendication 4 **caractérisé en ce que** le corps mobile (3) est pourvu d'un passage libre réservé à l'extraction, permettant de faire communiquer l'extraction du corps principal (7) aux canaux périphériques d'aspiration (31) du cylindre d'induction (2) de sorte que l'extraction des gaz anesthésiants résiduels est toujours active quelle que soit la position du cylindre d'induction (2).

6. Dispositif selon l'une quelconque des revendications 1 à 5 **caractérisé en ce qu'**une extrémité du corps mobile (3) est pourvu d'une camé (33) permettant la commande d'ouverture ou de fermeture du passage des gaz anesthésiants selon que le cylindre d'induction (2) est en position verticale ou en position horizontale.

7. Dispositif selon l'une quelconque des revendications 1 à 6 **caractérisé en ce que** le corps mobile (3) est pourvu d'un clapet (6) qui, maintenu en contact avec le corps principal (7) à l'aide d'un ressort (34), désactive le passage des gaz anesthésiants du corps principal (7) vers le cylindre d'induction (2), lorsque le cylindre d'induction est amené en position horizontale depuis la position verticale.

8. Dispositif selon l'une quelconque des revendications 1 à 7 et 6 **caractérisé en ce que** le corps mobile (3) est pourvu d'un clapet (6) qui, décollé du corps principal (7) par la came (33), active le passage des gaz anesthésiants du corps principal (7) vers le cylindre d'induction (2), lorsque le cylindre d'induction est amené en position verticale depuis la position horizontale.

9. Dispositif selon l'une quelconque des revendications 1 à 8 et 2 **caractérisé en ce que** la partie fixe appelée corps principal (7) du système d'induction directe de gaz anesthésiant dispose de deux perçages dont l'un est relié aux circuits d'extraction des deux parties mobiles (2,3) puis au circuit coaxial allant vers l'unité d'aspiration des gaz anesthésiants résiduels via le système des prises male/femelle de raccordement à double obturation (4, 5) connecté et l'autre reliant les circuits d'induction de gaz anesthésiants des deux parties mobiles (2, 3) au circuit coaxial provenant du générateur de gaz anesthésiant via le système des prises male/femelle de raccordement à double obturation (4, 5) connecté.

10. Dispositif selon l'une quelconque des revendications 1 à 9 **caractérisé en ce que** la cellule d'isolement transférable comporte trois parties amovibles (10, 11, 16) s'adaptant au corps principal (7) lui-même équipé d'une molette de réglage (13), l'ensemble étant supporté par le bâti support (1) d'où il peut en être ôté afin d'être transféré vers une unité d'acquisition de données en imagerie médicale.

11. Dispositif selon l'une quelconque des revendications 1 à 9 et 10 **caractérisé en ce que** la cellule d'isolement transférable comprend , outre ses trois parties amovibles, un corps principal (7) avec deux perçages communiquant d'un côté avec les canaux (56) du profilé extrudé) du lit (10) lorsque celui-ci est emboîté dans un tunnel de maintien (11), lui-même emboîté dans le corps principal (7), et de l'autre côté à un système de réchauffe des gaz froids extrait (14, 17, 61).

12. Dispositif selon la revendication 11 **caractérisé en ce que** le premier perçage (23), relié à l'entrée (64) d'un échangeur thermique (61) est réservé à l'extraction des gaz froids provenant des canaux (56) du lit (10) à l'aide d'une pompe (14) et **en ce que** le deuxième perçage (22), relié à la sortie (63) d'un échangeur thermique (61) est réservé à la restitution des gaz réchauffés vers les canaux (56) du lit (10).

13. Dispositif selon la revendication 12 **caractérisé en ce que** l'échangeur thermique (61), constitué de matériaux amagnétiques et inséré dans le corps principal (7), constitue un système de traitement des fluides permettant l'admission d'un fluide froid et sa circulation dans un espace de réchauffe (60) de sorte que le fluide sera restitué en sortie (63) à la température voulue.

14. Dispositif selon l'une quelconque des revendications 1 à 13 et 10 **caractérisé en ce que** la première partie amovible appelée lit (10) de la cellule d'isolement transférable qui s'emboîte ou se déboîte d'un tunnel de maintien (11), dispose de repères gradués (51, 53), et de barres d'oreilles (50) pour un positionnement reproductible.

15. Dispositif selon l'une quelconque des revendications 11 à 14 **caractérisé en ce que** le lit (10) est réalisé en profilé extrudé en arc de cercle, de faible épaisseur et diamètre adapté, à la taille de l'animal, et **en ce qu'**il dispose de canaux (56) longitudinaux, de griffes (54) sur ses bords externes de sorte que des couples support (55) pour accessoires médicaux pourront s'y clipper.

16. Dispositif selon l'une quelconque des revendications 1 à 15 et 11 **caractérisé en ce que** le tunnel de maintien (11) complètement emboîté dans le corps principal (7) est agencé pour actionner un clapet (6) qui permet le passage des gaz anesthésiants.

17. Dispositif selon l'une quelconque des revendications 11 à 16 **caractérisé en ce que** le tunnel de maintien (11) s'emboîtant ou se déboîtant du corps principal (7) de la cellule d'isolement transférable , est munie d'un cône (19) et d'une pièce de dent (52) et dans le prolongement duquel existe deux canaux (18, 20) dont l'un (18) équipé d'un bouchon perforable (12) permet l'accès de l'extérieur vers l'intérieur de la cellule d'isolement transférable et l'autre (20) le passage des gaz anesthésiants délivrés en sortie de cône (19).

18. Dispositif selon l'une quelconque des revendications 11 à 17 **caractérisé en ce que** le tunnel de maintien (11) est pourvu de canaux périphériques d'extraction (21) permettant l'aspiration les gaz anesthésiants résiduels et est orientable en inclinaison suivant son axe longitudinal.

19. Dispositif selon l'une quelconque des revendications 1 à 18 et 10 **caractérisé en ce que** la cellule d'isolement transférable dispose d'une troisième partie amovible appelée contenant externe transparent (16) qui isole complètement son contenu de l'air ambiant lorsque ledit contenant externe transparent (16) est vissé à la molette de réglage (13) de puissance d'aspiration des gaz pollués; ladite molette de réglage (13) étant montée en liaison pivot sur le corps principal (7) avec rotation limitée à 90 ° par rapport à l'axe longitudinal de la cellule d'isolement transférable.

20. Dispositif selon la revendication 19 **caractérisé en ce que** la molette de réglage (13), comporte deux positions de réglage à savoir une position ouverte qui permet une extraction maximale de par l'obstruction d'une prise d'air (41), et une position fermée qui permet une extraction minimale de par l'ouverture de la prise d'air (41) des gaz anesthésiants de surpression, de sorte que la pression interne dans la cellule d'isolement transférable sera très légèrement positive grâce à l'apport des gaz anesthésiants.

## Patentansprüche

1. Vorrichtung, die für Anwendungen bestimmt ist, welche mit Untersuchungstechniken in der medizinischen Bildgebung eines kleinen Labortiers, wie der Magnetresonanzbildgebung, der Röntgen-Mikrocomputertomographie, den szintigraphischen Auswertungstechniken mittels Single-Photon- und Biphoton-Emittern, der optischen Bildgebung, verbunden sind, wobei die Vorrichtung umfaßt:
- ein System zum direkten Einlassen von Narkosegas im Bereich seiner Schnauze, wodurch sein Fell vor dem Narkosegas geschützt wird, und das sein Vorbetäuben ermöglicht, wobei das System zum direkten Einlassen von Narkosegas zwei bewegliche Teile (2, 3) und einen als Hauptkörper (7) bezeichneten festen Teil, der mit einem Gestell (1) fest verbunden ist, umfaßt, wobei der eine der beweglichen Teile (2), der als Einlaßzylinder bezeichnet wird, sich durch Ausrücken von dem zweiten beweglichen Teil (3) trennen kann, und
- eine transportierbare Isolationszelle, die das sehr präzise und reproduzierbare Positionieren des Körpers des Tiers sowie ihr Isolieren gegenüber der Umgebungsluft, wenn sie geschlossen ist, sicherstellt, wobei die transportierbare Isolationszelle das Halten des Tiers unter längerer Narkose, sein Aufwärmen, seine medizinische Behandlung über das Blut oder den Atemtrakt ermöglicht, so daß die das in Behandlung befindliche Tier enthaltende transportierbare Isolationszelle, die offen oder geschlossen verwendet wird, manuell oder mittels Robotersystem bei Bildgeräten für die Aufnahme medizinischer Bilder eingesetzt werden kann,
**dadurch gekennzeichnet, daß** der zweite bewegliche Teil (3), der als beweglicher Körper bezeichnet wird, an dem Hauptkörper (7) drehbar angebracht ist, so daß der Einlaßzylinder (2) von einer horizontalen Position in eine vertikale Position schwenken kann, wobei der zweite bewegliche Teil (3) mit einem gesteuerten und für das Einlassen von Narkosegas vorgesehenen Durchgang versehen ist, der ermöglicht, den Narkosegaseinlaß des Hauptkörpers (7) mit einem mittleren Kanal (30) des Einlaßzylinders (2) zu verbinden, so daß das Einlassen von Narkosegas beim Übergang des Einlaßzylinders (2) von der horizontalen Position in die vertikale Position aktiviert wird, wobei die transportierbare Isolationszelle einen als Bett (10) bezeichneten ersten lösbaren Teil umfaßt, der als stranggepreßtes Profil ausgebildet ist, das ermöglicht, Längskanäle (56) in seine Dicke zu integrieren, die für den Durchlaß von verschiedenen zu befördernden Fluiden, wie Narkosegasen und/oder einem Wärmeträger für die Thermoregulation des auf dem Bett (10) enthaltenen Tiers, verwendet werden.

2. Vorrichtung nach Anspruch 1, **dadurch gekennzeichnet, daß** das System zum direkten Einlassen von Narkosegas und die transportierbare Isolationszelle Zubehörteile eines Narkosegaserzeugers und eines Systems zum Abziehen der verunreinigten Gase sind und hiermit mittels eines koaxialen Kreises verbunden sind, der die ungehinderte und unabhängige Zirkulation zweier Gase ermöglicht, von denen die eine für das Einlassen der Narkosegase und die andere für das Abziehen der verunreinigten Gase bestimmt ist.

3. Vorrichtung nach Anspruch 2, **dadurch gekennzeichnet, daß** das System zum direkten Einlassen von Narkosegas und die transportierbare Isolationszelle über einen weiblichen Verbindungsanschluß (4) mit Zweifachverschluß verfügen, der mit einem männlichen Verbindungsanschluß (5) mit Zweifachverschluß des koaxialen Kreises zu verbinden ist, so daß die ungehinderte und unabhängige Zirkulation von zwei Gasen erfolgt, wenn die beiden Anschlüsse verbunden sind, während sie unterbunden wird und das System zum direkten Einlassen und die transportierbare Isolationszelle isoliert, wenn die beiden Anschlüsse, der männliche (5) und der weibliche (4), voneinander getrennt sind.

4. Vorrichtung nach einem der Ansprüche 1 bis 3, **dadurch gekennzeichnet, daß** der erste bewegliche Teil (2) mit umfangseitigen Saugkanälen (31) versehen ist, die das ungehinderte Abziehen der restlichen Narkosegase ermöglichen.

5. Vorrichtung nach Anspruch 4, **dadurch gekennzeichnet, daß** der bewegliche Körper (3) mit einem für das Abziehen vorgesehenen freien Durchgang versehen ist, der ermöglicht, den Abzug des Hauptkörpers (7) mit den umfangseitigen Saugkanälen (31) des Einlaßzylinders (2) zu verbinden, so daß das Abziehen der restlichen Narkosegase unabhängig von der Position des Einlaßzylinders (2) stets aktiv ist.

6. Vorrichtung nach einem der Ansprüche 1 bis 5, **dadurch gekennzeichnet, daß** ein Ende des beweglichen Körpers (3) mit einem Nocken (33) versehen ist, der die Steuerung zum Öffnen oder zum Schließen des Durchgangs der Narkosegase ermöglicht, je nachdem, ob der Einlaßzylinder (2) sich in vertikaler Position oder in horizontaler Position befindet.

7. Vorrichtung nach einem der Ansprüche 1 bis 6, **dadurch gekennzeichnet, daß** der bewegliche Körper (3) mit einer Klappe (6) versehen ist, die - mit Hilfe einer Feder (34) mit dem Hauptkörper (7) in Kontakt gehalten - den Durchgang der Narkosegase von dem Hauptkörper (7) zu dem Einlaßzylinder (2) deaktiviert, wenn der Einlaßzylinder aus der vertikalen Position in die horizontale Position gebracht wird.

8. Vorrichtung nach einem der Ansprüche 1 bis 7 und 6, **dadurch gekennzeichnet, daß** der bewegliche Körper (3) mit einer Klappe (6) versehen ist, die - durch den Nocken (33) von dem Hauptkörper (7) abgehoben - den Durchgang der Narkosegase von dem Hauptkörper (7) zu dem Einlaßzylinder (2) aktiviert, wenn der Einlaßzylinder aus der horizontalen Position in die vertikale Position gebracht wird.

9. Vorrichtung nach einem der Ansprüche 1 bis 8 und 2, **dadurch gekennzeichnet, daß** der als Hauptkörper (7) des Systems zum direkten Einlassen von Narkosegas bezeichnete feste Teil über zwei Bohrungen verfügt, von denen die eine mit den Abzugskreisen der beiden beweglichen Teile (2, 3), dann mit dem koaxialen Kreis, der über das angeschlossene System der männlichen/weiblichen Verbindungsanschlüsse mit Zweifachverschluß (4, 5) zu der Einheit zum Absaugen der restlichen Narkosegase verläuft, verbunden ist, und wobei die andere die Kreise zum Einlassen von Narkosegasen der beiden beweglichen Teile (2, 3) mit dem koaxialen Kreis verbindet, der über das angeschlossene System der männlichen/weiblichen Verbindungsanschlüsse mit Zweifachverschluß (4, 5) von dem Narkosegaserzeuger kommt.

10. Vorrichtung nach einem der Ansprüche 1 bis 9, **dadurch gekennzeichnet, daß** die transportierbare lsolationszelle drei lösbare Teile (10, 11, 16) umfaßt, die auf den Hauptkörper (7) passen, welcher selbst mit einem Einstellrad (13) ausgestattet ist, wobei die Anordnung von dem Traggestell (1) getragen ist, von dem sie abgenommen werden kann, um zu einer Einheit zur Erfassung medizinischer Bildgebungsdaten transportiert zu werden.

11. Vorrichtung nach einem der Ansprüche 1 bis 9 und 10, **dadurch gekennzeichnet, daß** die transportierbare Isolationszelle außer ihren drei lösbaren Teilen einen Hauptkörper (7) mit zwei Bohrungen umfaßt, die einerseits mit den Kanälen (56) des stranggepreßten Profils des Betts (10) verbunden sind, wenn dieses in einen Haltetunnel (11) eingesteckt ist, der selbst in den Hauptkörper (7) eingesteckt ist, und andererseits mit einem System zum Erhitzen der abgezogenen kalten Gase (14, 17, 61) verbunden sind.

12. Vorrichtung nach Anspruch 11, **dadurch gekennzeichnet, daß** die erste Bohrung (23), die mit dem Eingang (64) eines Wärmetauschers (61) verbunden ist, für das Abziehen der aus den Kanälen (56) des Betts (10) kommenden kalten Gase mit Hilfe einer Pumpe (14) vorgesehen ist, und daß die zweite Bohrung (22), die mit dem Ausgang (63) eines Wärmetauschers (61) verbunden ist, für die Rückgabe der erhitzten Gase an die Kanäle (56) des Betts (10) vorgesehen ist.

13. Vorrichtung nach Anspruch 12, **dadurch gekennzeichnet, daß** der Wärmetauscher (61), der von unmagnetischen Materialien gebildet und in den Hauptkörper (7) eingesetzt ist, ein Fluidbehandlungssystem bildet, das das Zuführen eines kalten Fluids und dessen Zirkulation in einem Erhitzungsraum (60) ermöglicht, so daß das Fluid am Ausgang (63) mit der gewünschten Temperatur zurückgegeben wird.

14. Vorrichtung nach einem der Ansprüche 1 bis 13 und 10, **dadurch gekennzeichnet, daß** der als Bett (10) bezeichnete erste lösbare Teil der transportierbaren Isolationszelle, der in einen Haltetunnel (11) eingesteckt oder aus diesem ausgerückt ist, über Skalenmarkierungen (51, 53) und über Ohrenleisten (50) für ein reproduzierbares Positionieren verfügt.

15. Vorrichtung nach einem der Ansprüche 11 bis 14, **dadurch gekennzeichnet, daß** das Bett (10) als kreisbogenförmiges stranggepreßtes Profil mit geringer Dicke und einem an die Größe des Tiers angepaßten Durchmesser ausgebildet ist, und daß es über Längskanäle (56), über Klauen (54) an seinen Außenrändern verfügt, so daß Tragpaare (55) für medizinische Zubehörteile hieran festgeclipst werden können.

16. Vorrichtung nach einem der Ansprüche 1 bis 15 und 11, **dadurch gekennzeichnet, daß** der vollständig in den Hauptkörper (7) eingesteckte Haltetunnel (11) dazu eingerichtet ist, eine Klappe (6) zu betätigen, die den Durchgang der Narkosegase ermöglicht.

17. Vorrichtung nach einem der Ansprüche 11 bis 16, **dadurch gekennzeichnet, daß** der Haltetunnel (11), der in den Hauptkörper (7) der transportierbaren Isolationszelle eingesteckt oder aus diesem ausgerückt ist, mit einem Kegel (19) und einem Zahnteil (52) versehen ist und in dessen Verlängerung zwei Kanäle (18, 20) vorhanden sind, von denen der eine (18), der mit einem durchbohrbaren Verschluß (12) versehen ist, den Zugang von der Außenseite zum Innenraum der transportierbaren Isolationszelle und der andere (20) den Durchgang der am Ausgang des Kegels (19) gelieferten Narkosegase ermöglicht.

18. Vorrichtung nach einem der Ansprüche 11 bis 17, **dadurch gekennzeichnet, daß** der Haltetunnel (11) mit umfangseitigen Abzugskanälen (21) versehen ist, die das Absaugen der restlichen Narkosegase ermöglichen, und um seine Längsachse neigungsverstellbar ist.

19. Vorrichtung nach einem der Ansprüche 1 bis 18 und 10, **dadurch gekennzeichnet, daß** die transportierbare Isolationszelle über einen als transparenter Außenbehälter (16) bezeichneten dritten lösbaren Teil verfügt, der ihren Inhalt gegenüber der Umgebungsluft vollständig isoliert, wenn der transparente Außenbehälter (16) an das Rad zum Einstellen (13) der Leistung zum Absaugen der verunreinigten Gase geschraubt ist; wobei das Einstellrad (13) an dem Hauptkörper (7) mit einer auf 90° gegenüber der Längsachse der transportierbaren Isolationszelle begrenzten Drehung drehbar angebracht ist.

20. Vorrichtung nach Anspruch 19, **dadurch gekennzeichnet, daß** das Einstellrad (13) zwei Einstellpositionen aufweist, nämlich eine geöffnete Position, die aufgrund des Verschließens eines Lufteinlasses (41) ein maximales Abziehen ermöglicht, und eine geschlossene Position, die aufgrund des Öffnens des Lufteinlasses (41) ein minimales Abziehen der Überdrucknarkosegase ermöglicht, so daß der Innendruck in der transportierbaren Isolationszelle dank des Zuführens der Narkosegase sehr leicht positiv sein wird.

## Claims

1. A device dedicated to applications associated with techniques for medical imaging investigations of small laboratory animals, such as magnetic resonance imaging, X-ray micro-tomodensitometry, techniques using scintigraphy by single-photon and two-photon emitters, and optical imaging, the device comprising:
- a system for direct inducing anesthetic gas to the muzzle of the animal, thereby preserving its coat from the anesthetic gas and enabling pre-anesthesia of the animal, the system for direct induction of anesthetic gas comprising two moving portions (2, 3), and a stationary portion that is referred to as the main body (7) and that is secured to a structure (1), one of the moving portions (2), referred to as the induction cylinder, can be separated from the second moving portion (3) by disengagement; and
- a transferable isolation cell enabling the body of the animal to be positioned very accurately and reproducibly and enabling the animal to be isolated from ambient air when closed, said transferable isolation cell enabling the animal to be maintained under prolonged anesthesia, to be heated, and to be treated medically via the blood or respiration so that said transferable isolation cell containing the animal under treatment, which can be used open or closed, can be inserted manually or by means of a robotic system in images for acquiring medical images, the device being **characterized in that** the second moving portion (3), referred to as the moving body, is mounted via a pivot connection on the main body (7), such that the induction cylinder (2) can move from a horizontal position to a vertical position, the second moving body (3) being provided with a controlled passage reserved for induction of anesthetic gas, enabling the anesthetic gas induction of the main body (7) to communicate with a central channel (30) of the induction cylinder (2) in such a manner that anesthetic gas induction is activated when the induction cylinder (2) passes from the horizontal position to the vertical position, the transferable isolation cell comprises a first removable portion referred to as the bed (10), made from an extruded section member enabling longitudinal channels (56) to be incorporated in its thickness, which channels are used for passing various fluids that need to be conveyed, such as anesthetic gas or a heat-conveying fluid for regulating the temperature of the animal contained on the bed (10).

2. A device according to claim 1, **characterized in that** the system for direct induction of anesthetic gas and the transferable isolation cell are accessories of an anesthetic gas generator and of a system for extracting polluted gas, and are connected thereto by means of a coaxial circuit enabling two free and independent gas flows to be provided, one flow being dedicated to anesthetic gas induction, and the other flow being dedicated to extracting polluted gas.

3. A device according to claim 2, **characterized in that** the system for direct induction of anesthetic gas and the transferable isolation cell have respective double-closure female connectors (4) for connecting to a double-closure male connector (5) of the coaxial circuit such that the free and independent flows of two gases take place if the connectors are connected together, and are prevented with the direct induction system and the transferable isolation cell being isolated if the male and female connectors (4, 5) are disconnected.

4. A device according to any one of claims 1 to 3, **characterized in that** the first moving portion (2) is provided with peripheral suction channels (31) allowing residual anesthetic gas to be extracted freely.

5. A device according to claim 4, **characterized in that** the moving body (3) is provided with a free passage reserved for extraction, enabling the extraction of the main body (7) to communicate with the peripheral suction channels (31) of the induction cylinder (2), such that extraction of the residual anesthetic gas is always active regardless of the position of the induction cylinder (2).

6. A device according to any one of claims 1 to 5, **characterized in that** the end of the moving body (3) is provided with a cam (33) enabling the anesthetic gas passage to be opened or closed depending on whether the induction cylinder (2) is in the vertical position or in the horizontal position.

7. A device according to any one of claims 1 to 6, **characterized in that** the moving body (3) is provided with a valve (6) that, when held in contact with the main body (7) with the help of a spring (34), deactivates the anesthetic gas passage from the main body (7) to the induction cylinder (2) while the induction cylinder is moved from the vertical position to the horizontal position.

8. A device according to any one of claims 1 to 7 and 6, **characterized in that** the moving body (3) is provided with a valve (6) that, when spaced apart from the main body (7) by the cam (33), activates the passage of anesthetic gas from the main body (7) to the induction cylinder (2) while the induction cylinder is moved from the horizontal position to the vertical position.

9. A device according to any one of claims 1 to 8 and 2, **characterized in that** the stationary portion, referred to as the main body (7), of the system for direct induction of anesthetic gas has two holes, one of which is connected to the extraction circuits of the two moving portions (2, 3) and then to the coaxial circuit leading to the residual anesthetic gas suction unit via the system of double-closure male/female connectors (4, 5) while connected together, and the other connecting the anesthetic gas induction circuits of the two moving portions (2, 3) to the coaxial circuit coming from the anesthetic gas generator via the system of double-closure male/female connectors (4, 5) while connected together.

10. A device according to any one of claims 1 to 9, **characterized in that** the transferable isolation cell has three removable portions (10, 11, 16) fitted to the main body (7), itself fitted with an adjustment knob (13), the assembly being supported by the support structure (1) from which it can be removed in order to be transferred to a unit for acquiring medical imaging data.

11. A device according to any one of claims 1 to 9 and 10, **characterized in that** the transferable isolation cell comprises, in addition to its three removable portions, a main body (7) having two holes communicating at one end with the channels (56) of the extruded section member of the bed (10) when the bed is engaged in the holder tunnel (11), itself engaged in the main body (7), and at the other end with a system for heating the extracted cold gas (14, 17, 61).

12. A device according to claim 11, **characterized in that** the first hole (23) connected to the inlet (64) of a heat exchanger (61) is reserved for extracting cold gas coming from the channels (56) of the bed (10) with the help of a pump (14), and **in that** the second hole (22), connected to the outlet (63) of a heat exchanger (61) is reserved to restoring the heated gas to the channels (56) of the bed (10).

13. A device according to claim 12, **characterized in that** the heat exchanger (61) made of non-magnetic materials and inserted in the main body (7), constitutes a fluid processor system enabling a cold fluid to be admitted and to flow in a heater space (60) so that the fluid is returned at the outlet (63) at the desired temperature.

14. A device according to any one of claims 1 to 13 and 10, **characterized in that** the first removable portion, referred to as a bed (10), of the transferable isolation cell that is engaged or disengaged from a holder tunnel (11), possesses graduations (51, 53), and lug bars (50) to enable positioning to be reproducible.

15. A device according to any one of claims 11 to 14, **characterized in that** the bed (10) is made from an extruded section member in the shape of a circular arc, of small thickness and of diameter matching the size of the animal, and **in that** it has longitudinal channels (56) and strips (54) at its outer edges so that pairs of supports (55) for medical accessories can be clipped thereto.

16. A device according to any one of claims 1 to 15 and 11, **characterized in that** the holder tunnel (11) when fully engaged in the main body (7) is arranged to actuate a valve (6) that allows anesthetic gas to pass.

17. A device according to any one of claims 11 to 16, **characterized in that** the holder tunnel (11) engaging with or disengaging from the main body (7) of the transferable isolation cell is provided with a cone (19) and with a tooth piece (52), and has two channels (18, 20) in line therewith, one of the channels (18) being provided with a perforatable stopper (12) giving access from the outside to the inside of the transferable isolation cell, and the other channel (20) allowing anesthetic gas to pass as delivered at the outlet from the cone (19).

18. A device according to any one of claims 11 to 17, **characterized in that** the holder tunnel (11) is provided with peripheral extraction channels (21) enabling residual anesthetic gas to be sucked out and is orientable in inclination about its longitudinal axis.

19. A device according to any one of claims 1 to 18 and 10, **characterized in that** the transferable isolation cell possesses a third removable portion, referred to as a transparent outer container (16), that completely isolates the content thereof from ambient air when said transparent outer container (16) is screwed to the knob (13) for adjusting the power with which polluted gas is sucked out; said adjustment knob (13) being mounted via a pivot connection on the main body (7) with pivoting restricted to 90° relative to the longitudinal axis of the transferable isolation cell.

20. A device according to claim 19, **characterized in that** the adjustment knob (13) has two adjustment positions, namely an open position that maximizes extraction by obstructing the air intake (41), and a closed position that minimizes extraction by opening the air intake (41) for anesthetic gas under pressure, such that the internal pressure within the transferable isolation cell is very slightly positive as a result of the delivery of anesthetic gas.
